# EUROPEAN PATENT APPLICATION

(11) **EP 3 391 859 A1**
(43) Date of publication of application: **24.10.2018**
(21) Application number: 18168400.2
(22) Date of filing: 20.04.2018
(51) Int. Cl.: A61F 2/44, A61B 17/70, A61F 2/30

(54) **FACET JOINT IMPLANTS SYSTEM**

(30) Priority: 21.04.2017 PL 42135817
(71) Applicant: "CHM" Spolka Z Organiczona Odpowiedzialnoscia, 16-061 Juchowiec Koscielny (PL)
(72) Inventor: Sobolewski, Andrzej, 15-057 Bialystok (PL); Zarzycki, Daniel, 34-501 Zakopane (PL); Murawski, Zbigniew, 15-034 Bialystok (PL); Prokopiuk, Marcin, 15-361 Bialystok (PL)
(74) Representative: Bury, Marek

(57) **Abstract**

A facet joint implants system comprising the first implant (1) adapted to be attached to the first articular process, and the second implant (3) adapted to be attached to the second articular process. The system comprises also a sliding insert (2) adapted to be placed between the first implant (1) and the second implant (3). The first surface (2A) of the sliding insert (2) is complementary to a contact surface (1A) of the first implant (1) and the second surface (2B) of the sliding insert (2) is complementary to a contact surface (3A) of the second implant (3). The first surface (2A) of the sliding insert (2) is at least partially spherical, and the second surface (2B) of the sliding insert (2) is substantially planar.

## Description

The present invention concerns a facet joint implants system, that is used in a surgical treatment of the spine and that replaces degenerated articular facets of articular processes of the vertebrae.

The main cause of a chronic pain of the spine are spinal overload syndromes. They are characterized by long-lasting, often recurrent ailments of a variable nature. These ailments are closely related to degenerative changes in the spine, and in particular, with lesions involving facet (zygapophysial) joints.

A single facet joint is formed by inferior and superior articular processes of two adjacent vertebrae, covered with an articular cartilage and surrounded by a joint capsule. The facet joint and the joint capsule that surrounds the joint are strongly innervated by nerve branches from the spinal roots.

Degenerative changes result in a loss of articular cartilage and uncovering of subchondral bone which leads to the severe pain due to friction of degenerated articular facets against each other. The current treatment methods of degenerative changes are mainly focused on the removal of pain. These include but are not limited to physiotherapy, pharmacological treatment, surgical procedures to stiffen or relieve the level of the spine on which the changes occur. Unfortunately, all these treatments do not restore mobility in the facet joint.

The solution to this problem is the arthroplasty which allows for pain removal and restoration of mobility in the zygapophysial joint by replacing the damaged articulating surfaces with an implant.

An American patent application no. US20030191532 discloses an embodiment that requires the inferior and superior articular processes to be removed and replaced by prostheses. The disclosed prosthesis comprises a bolt inserted into a vertebral pedicle and a prosthesis head. Both elements function inseparably. As a variant of the disclosed embodiment, a screw fastener functions as the bolt that is inserted into the vertebral pedicle through the prosthesis head fixing the head to the bone structures.

The embodiment presented in the US patent application no. US20080221622 relates to the design of a prosthesis, the use of which requires the articular processes to be removed and replaced by multi-element prostheses. An inferior prosthesis comprises a frame made of articulated connectors and rods, prosthesis heads attached to the frame and screws anchoring the frame in the vertebral pedicles. Additionally, the frame is anchored in a spinous process. The superior prostheses are made of a screw inserted into a vertebral pedicle, a prosthesis head and an articulated connector that joins the screw with the head.

An American patent application no. US20100241164 discloses a construction distinguished by locking an implant with use of surgical threads. The implantation place and insertion are the same as for an embodiment described in an application no. US20050049705.

The American patent application no. US20050049705 presents prostheses of articular facets of the articular processes. The fixation of the prosthesis of the superior articular process is performed by a conical spike (being an integral part of the prosthesis) being pressed into bone structures (joint surface).The prosthesis of the inferior articular process is attached with use of a screw which is screwed into the prosthesis and passes through a vertebral arch and spinous process.

An alternative embodiment of a facet joint implant is provided in an American patent no. US9017389. Two small discs made of PEEK plastic are inserted directly into a zygapophysial joint. The implants are positioned with use of sharp anchoring elements located on one side of the implant which are pressed into the articular faces as a result of the forces existing in the joint.

Constructions known from the prior art can therefore be divided into two types of embodiment designs of such implants.

The use of the first type of the design requires complete removal of the articular processes. They are replaced by implants that reflect the anatomical functions of the facet joint. This is a total joint arthroplasty. Such procedure results in damage of bone structures (vertebral pedicles, articular processes), muscles and ligaments which hinders rehabilitation and is associated with an increased risk of complications.

In the case of the second type of the implant construction, only the damaged articulating surfaces are replaced with implants which allows minimally invasive insertion of the implants, slight interference with bone and muscle structures, faster healing of postoperative wounds, restoration of full joint function, faster patient's recovery and their return to daily activities. The problems are however, the incomplete range of mobility, insufficient fixation of the implant and the rate of tribological wear.

The aim of the invention is to solve these problems by providing a facet joint implants system.

The facet joint implants system containing the first implant adapted to be attached to the first articular process and the second implant adapted to be attached to the second articular process. The system includes also a sliding insert adapted to be placed between the first implant and the second implant. The first surface of the sliding insert is complementary to the contact surface of the first implant and the second surface of the sliding insert is complementary to the contact surface of the second implant. The first surface of the sliding insert is at least partially of a spherical shape and the second surface of the sliding insert is substantially planar. Thanks to such design of the implants system, a full range of mobility is achieved while the number of tribological products in the functioning system of implants is reduced.

Advantageously, the first surface of the sliding insert is convex. Then the complementary surface of the implant is concave. Alternatively, the first surface of the sliding insert is concave and the complementary surface of the implant is convex.

Advantageously embodiment, at least one implant is provided at its top with a protrusion with at least one mounting hole. Additionally, at least one mounting hole is preferably inclined with respect to the back surface of at least one implant at an angle ranging from 0° to 60° which ensures that the implant is pressed up against an articular process. The risk of implant loosening can be reduced if the implant is provided with at least two mounting holes inclined with respect to each other at an angle ranging from 0° to 90°. Preferably, the implants system comprises bone screws, and the holes interact better with the screws when provided with a thread that fits the locking thread of the bone screws.

Advantageously, at least one implant is, at least on a part of the perimeter of the contact surface, provided with a flange forming an undercut with the contact surface, while the sliding insert is provided, at least on a part of the perimeter, with a ring that fits the inside of the undercut. Thanks to this, the insert is connected to the implant while maintaining its sliding property with respect to this implant. Such configuration simplifies the implantation of the implants system.

Advantageously, at least one implant is provided on its back surface with anchoring elements which improve the stability of the implant attachment. Additional stabilization can be obtained by providing the implant in its back part with an undercut adapted to receive an articular process. A partial resection of the process is acceptable so that it fits better the undercut. The optional covering of the back surface of the implant and/ or undercut with a porous coating facilitates bone ingrowth and increases the strength of the implant-to-process connection.

Advantageously, the first implant is a superior implant being adapted to cooperate with an superior articular process, and the second implant is an inferior implant being adapted to cooperate with an inferior articular process. Alternatively, the first implant is an inferior implant being adapted to cooperate with an inferior articular process, while the second implant is a superior implant being adapted to cooperate with a superior articular process.

Advantageously, the sliding insert is made of a material comprising at least one material selected from the group comprising of biocompatible polymers, biocompatible ceramic materials and biocompatible metal materials. These materials are characterized by mechanical strength and low coefficient of friction.

In an advantageous embodiment, at least one implant is made of a material comprising a material selected from the group consisting of biocompatible polymers, biocompatible ceramics, and biocompatible metal materials.

The exemplary embodiment of the invention is described below in detail with reference to the attached drawings in which Fig. 1 presents a simplified side view of the spine, Fig. 2 presents the posterior (b) and lateral (a) view of the spine section at L3-L4 level with placed facet joint implants system, Fig. 3 presents the exploded view of the facet joint implants system, Fig. 4 presents the superior implant 1 in the anterior (a), lateral (b), posterior (c), bottom (d), top (e) and cross-sectional (f) view, Fig. 5 presents the sliding insert 2 in the front view (a), side half-view (b), posterior view (c), Fig. 6 presents the inferior implant 3 in the front (a), side (b), posterior (c), top (d) view, and Fig. 7 presents the inferior implant 1 with mounted sliding insert in the front (a) and cross- sectional (b) view.

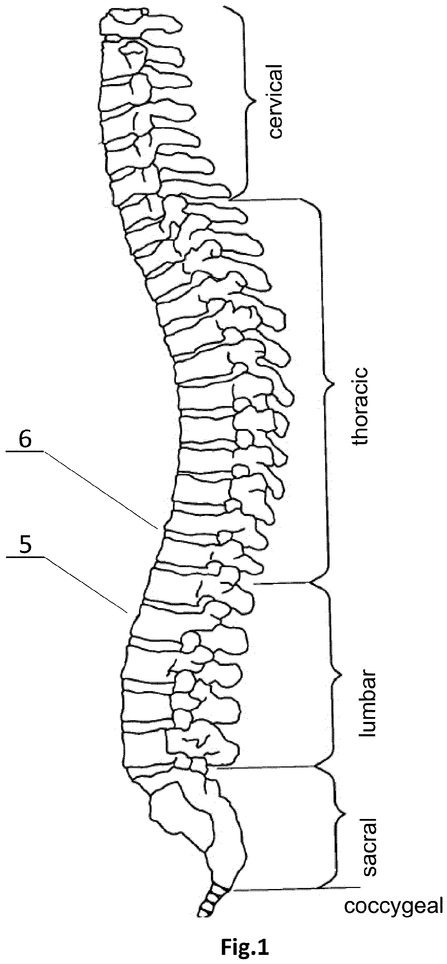

The human spine is formed by vertebrae 5 divided into 5 sections: cervical, thoracic, lumbar, sacral and coccygeal, as shown in Fig. 1. In the sacral and coccygeal sections, the vertebrae are fused together to form the sacral and caudal bone. The remaining vertebrae lying in the cervical, thoracic and lumbar sections are separated from each other by intervertebral discs 6. The discs, due to their flexibility, absorb the forces acting on the spine and allow for mobility between adjacent vertebrae.

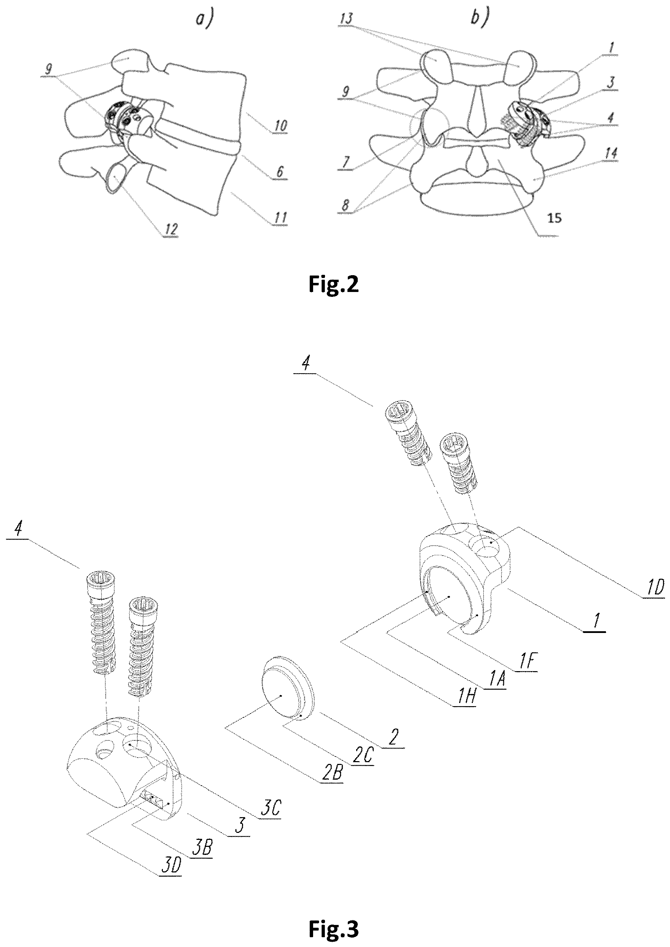

An additional element stabilizing the mobility of the spine and transferring some of the forces acting on it are facet (zygapophysial) joints 7. The zygapophysial joints 7, presented in Fig. 2, are situated in a place where the articulating surfaces of the inferior 8 and superior 9 articular processes of adjacent vertebrae, proximal 10 and distal 11 are in contact. Articular facets 12 and 13 are covered with articular cartilage. Degenerative changes and mechanical injuries cause the disappearance of articular cartilage and destruction of articular facets resulting in severe pain and limitation or complete disappearance of mobility in the facet joints. This can be cured using the implants system that includes superior 1 and inferior 3 implant fixed with bone screws 4.

The implants system is presented in detail in the exploded view in Fig. 3. The purpose of the system is to replace a degenerated joint and take over its anatomical functions. This system includes the first implant 1 being the superior implant, the second implant 3 being the inferior implant, the sliding insert 2 positioned between the implants, and the bone screws 4 for fixing the superior 1 and inferior 3 implant to the bone.

The first and second implants are made of a biocompatible PEEK CFR polymer reinforced with carbon fibers. The sliding insert 2 is made of biocompatible cobalt alloy, while bone screws of biocompatible type of titanium. It should be noted that, alternatively, these elements can be made using materials selected from the group consisting of biocompatible polymers used for orthopaedic implants, biocompatible ceramics, biocompatible metal materials. Only in the case of bone screws, biocompatible metal materials are particularly desirable.

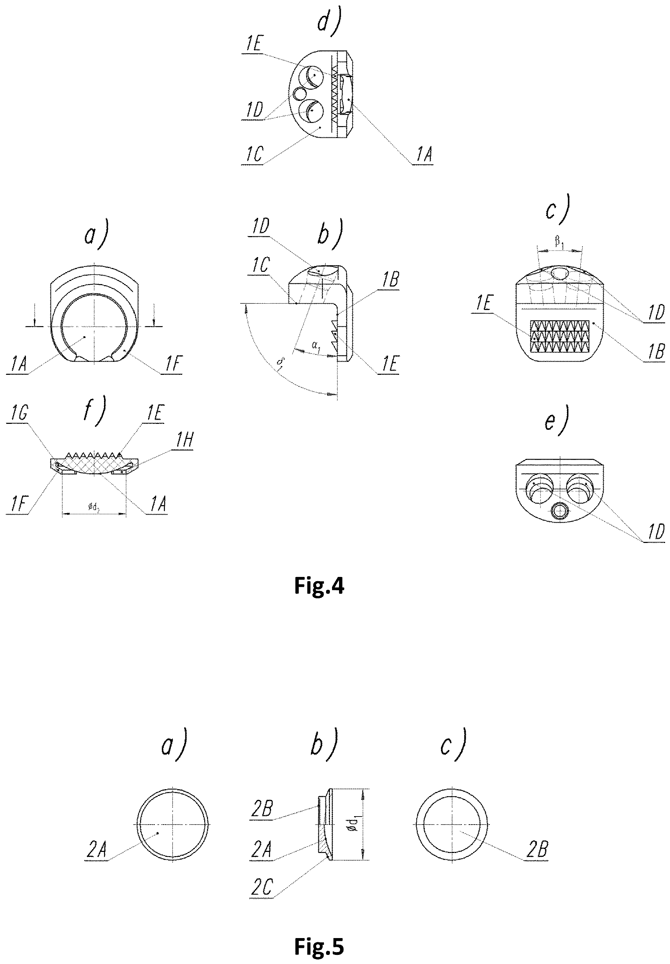

The superior implant 1 presented in Fig. 4 replaces the articular facet 12 of the inferior articular process 8 of the proximal vertebra 10 presented in Fig. 2. The implant is positioned on the articular process by locating the back surface 1B of the implant against the articular facet 12 of the articular process, and the surface 1C on the surface 14 being the outer surface of the inferior articular process 8 which passes into the lamina of the vertebral arch 15. The surfaces 1B and 1C are aligned in relation to each other at an angle δ₁ which corresponds to the angle between the articular facet 12 and the outer surface 14 of the inferior articular process. In this embodiment, δ₁ is 90°, but it can range from 45° to 135°. In order to better position the implant in the bone structures, a small resection of the articular facet 12 and the outer surface 14 is acceptable. The implant is attached to the articular process with use of the bone screws 4 presented in Fig. 3. The screws are inserted through the holes 1D located in the upper part of the superior implant 1 into the inferior articular process 8 through its outer surface 14. The holes 1D are inclined to the surface 1B at an angle α1 = 20° so that the surfaces 1B and 1C are simultaneously pressed against the bone structures of the inferior articular process 8 when introducing the bone screws 4. A similar effect can also be obtained for other values of angle α1 in the range from 0° to 60°. The holes 1D can be positioned at an angle β₁ = 12° in relation to each other which reduces the risk of implant loosening. The values of β₁ in the range from 0° to 90° are permissible. Additionally, anchoring elements 1E in the shape of pyramids or cones are provided on the back surface 1B to improve the stability of the implant. It is also permissible to use anchoring elements in the shapes of other narrowing bodies, not necessarily monotonically, as a function of the distance from the surface 1B and equipped with sharp edges. An additional improvement of the fixation of the superior implant 1 is achieved by covering the surfaces 1B and 1C with a porous coating. This facilitates bone ingrowth, providing additional stabilization of the implant.

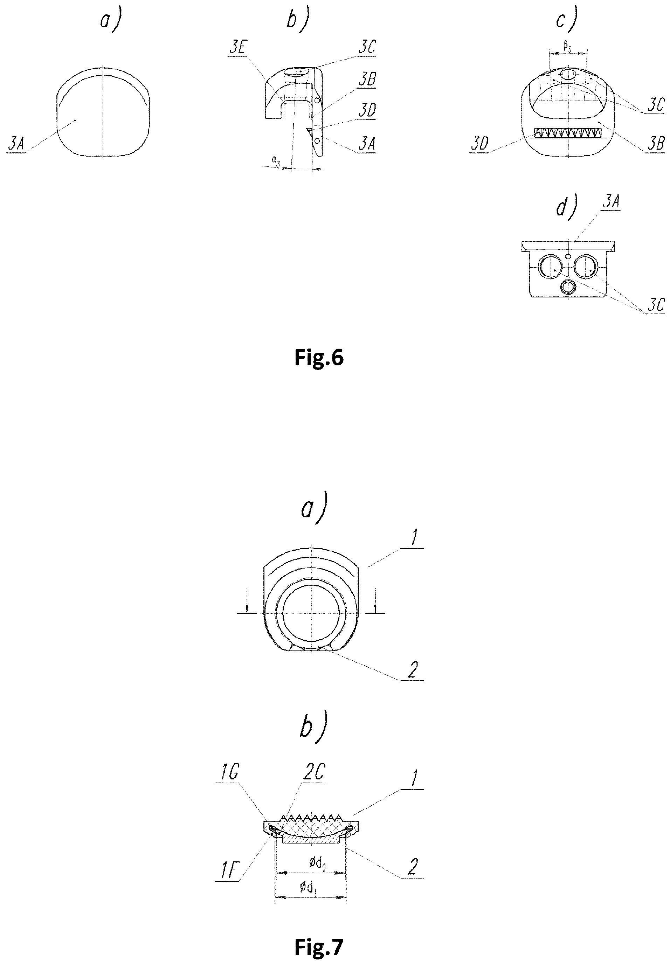

The inferior implant 3 presented in Fig. 6 replaces the articular facet 13 of the superior articular process 9 of the distal vertebra 11. The implant is positioned on the articular process by locating the back surface 3B of the inferior implant 3 against the articular facet 13 of the articular process. In the upper part, the articular process is inserted into the undercut 3E that is performed in the upper part of the implant. A small resection of the upper part of the articular process is acceptable for a better fit to the undercut 3E. The inferior implant 3 is attached to the articular process 9 with use of the bone screws 4 as presented in Fig. 3. The screws are inserted through the holes 3C located in the upper part of the inferior implant 3 into the superior articular process 9. The holes 3C are inclined in relation to each other at an angle β₃ = 6° and in relation to the surface 3B at angle α₃ = 0° which reduces the risk of implant loosening. Other values of these angles are permissible, but to ensure that the risk of implant loosening is reduced, α₃∈(0°,60°) and β₃∈(0°,90°) should be preserved. Additional stability is achieved by providing surface 3B with anchoring elements 3D in the shape of pyramids or cones. It is also permissible to use anchoring elements in the shapes of other narrowing bodies, not necessarily monotonically, as a function of the distance from the surface 3B and equipped with sharp edges. An additional improvement of the fixation of the inferior implant 3 is achieved by covering the surface 3B and the surfaces forming the undercut 3E with a porous coating to facilitates bone ingrowth.

In the case of two-piece implants systems in which an inferior implant replaces an inferior articular facet and a superior implant replaces a superior articular facet, the range of mobility in a facet joint, i.e. the linear and angular displacement between the joints, depends on the interaction of a spherical surface of one of the implants with a planar or curved surface of the other implant. In order to ensure the full range of this movement, it is required to provide a small, almost point-like area of contact of the implant surfaces, which in turn results in the occurrence of large stresses and local elastic deformations of the materials. This leads to rapid destruction and wear of interacting surfaces of implants and the formation of excessive amount of tribological products. A solution to this problem in the implants system of the present embodiment is the introduction, between the inferior 1 and superior 3 implants, of the sliding insert 2 as presented in Fig. 5. The sliding insert 2 is of a disc shape with a diameter d₁ and has a surface 2A constituting substantially a concave section of the sphere whose radius of curvature corresponds to the radius of curvature of the surface 1A of the superior implant 1 having the shape of a convex section of the sphere. The diameter d₁ is, in most applications, in the range from 5 mm to 15 mm.

The spherical surface 2A of the sliding insert 2 is in contact with the surface 1A of the superior implant 1 during normal functioning of the system. The spherical surface 2A in the present embodiment is concave. Alternatively, the surface may be convex but then the first implant with a concave contact surface should be used. These surfaces are always complementary.

On its other side, the sliding insert 2 has a substantially planar surface 2B which cooperates with the planar surface 3A of the superior implant 3. These surfaces are also complementary.

In the compound implants system placed in the patient's joint, the sliding insert 2 can move against the contact surface 1A of the first superior implant 1 and the contact surface 3A of the second, inferior implant 3.

Alternatively, a system can be performed in which the contact surface of the inferior implant is spherical and of the superior implant - planar.

Easier assembly of the system can be achieved by having the sliding insert 2 been attached to at least one of the implants - inferior 1 or superior 3. An example of such attachment is presented in Fig. 7. In this example, the sliding insert is attached to the superior implant 1. The sliding insert is equipped with the ring 2C that is inserted into the undercut 1G that results from surrounding the surface 1A of the superior implant 1 with the flange 1F as presented in Fig. 4f. The diameter d₁ of the ring 2C of the sliding insert 2 is larger than the diameter d₂ of the toroidal surface 1H formed by the flange 1F, so that the sliding insert 2 is joined with the superior implant 1 but can move along the surface 1A. If the flange 1F is open at a portion of the perimeter of the surface 1A, it is possible then to insert the sliding insert 2 through that opening by "press-fitting" the ring 2C into the inner surface 1H of the undercut 1G.

## Claims

1. A facet joint implants system comprising a first implant (1) adapted to be attached to a first articular process, and a second implant (3) adapted to be attached to a second articular process, **characterized in that** the system comprises also a sliding insert (2) adapted to be placed between the first implant (1) and the second implant (3), wherein a first surface (2A) of the sliding insert (2) is complementary to a contact surface (1A) of the first implant (1) and a second surface (2B) of the sliding insert (2) is complementary to a contact surface (3A) of the second implant (3), wherein the first surface (2A) of the sliding insert (2) is at least partially spherical, and the second surface (2B) of the sliding insert (2) is substantially planar.

2. The implants system according to claim 1, **characterized in that** the first surface (2A) of the sliding insert (2) is convex.

3. The implants system according to claim 1, **characterized in that** the first surface (2A) of the sliding insert (2) is concave.

4. The implants system according to claim 1 or 2 or 3, **characterized in that** at least one implant (1,3) at its top is provided with a protrusion having at least one mounting hole (1D, 3C).

5. The implants system according to claim 4, **characterized in that** at least one mounting hole (1D, 3C) is inclined at an angle (α₁, α₃) with respect to the back surface (1B, 3B) of at least one implant (1,3), ranging from 0° to 60°.

6. The implants system according to claim 4 or 5, **characterized in that** at least one implant is provided with at least two mounting holes (1D, 3C) inclined with respect to each other at an angle in the range of 0° to 90°.

7. The implants system according to claim 4 or 5 or 6, **characterized in that** at least one hole (1D, 3C) is provided with a thread.

8. The implants system according to any of claims from 4 to 7, **characterized in that** it comprises at least one bone screw.

9. The implants system according to any of the claims from 1 to 8, **characterized in that** at least one implant (1) has at least a portion of the perimeter of the contact surface (1A) provided with a flange (1F) forming with the contact surface (1A) an undercut (1G), and the sliding insert (2) is at least on the part of the perimeter equipped with a ring (2C) fitting to the inside of the undercut (1G).

10. The implants system according to any of the claims from 1 to 9, **characterized in that** at least one implant (1,3) is provided on its back surface (1B, 3B) with anchoring elements (1E, 3D).

11. The implants system according to any of the claims from 1 to 10, **characterized in that** at least one implant (3) is provided with an undercut (3E) adapted to receive an articular process.

12. The implants system according to any of the claims from 1 to 11, **characterized in that** the back surface of at least one implant (1B, 3B) is at least partially covered by a porous coating.

13. The implants system according to any of the claims from 1 to 12, **characterized in that** the first implant (1) is the superior implant being adapted to interact with the superior articular process, and the second implant (3) is the inferior implant being adapted to interact with the inferior articular process.

14. The implants system according to any of the claims from 1 to 12, **characterized in that** the first implant (1) is the inferior implant being adapted to interact with the inferior articular process, and the second implant (3) is the superior implant being adapted to interact with the superior articular process.

15. The implants system according to any of the claims from 1 to 14, **characterized in that** the sliding insert (2) and at least one implant are made of a material comprising a material selected from a group comprising biocompatible polymers, biocompatible ceramics and biocompatible metal materials.
